# EUROPEAN PATENT APPLICATION

(11) **EP 1 480 154 A2**
(43) Date of publication of application: **24.11.2004**
(21) Application number: 04076449.0
(22) Date of filing: 18.05.2004
(51) Int. Cl.: G06F 19/00

(54) **A comprehensive searchable medical record system supporting healthcare delivery and experiment**

(30) Priority: 19.05.2003 US 471651; 13.05.2004 US 844752
(71) Applicant: SIEMENS CORPORATE RESEARCH, INC., Princeton, New Jersey 08540 (US)
(72) Inventor: Datta, Debarshi, Norht Brunswick, NJ 08902 (US); Owens, Steven, Denville, NJ 07434-9601 (US); Tarbox, Lawrence, New Egypt, NJ 08533 (US); Zahlmann, Gudrun Dr., 92318 Neumarkt (DE)
(74) Representative: Morgan, Marc

(57) **Abstract**

Among the many potential embodiments is a searchable electronic medical record system comprising: an interface for receiving data representing a search item; at least one repository associating a subject identifier with medical information specific to a particular subject comprising: genomic information, clinical data, and laboratory test results; and a search processor for searching said at least one repository to find medical information comprising said search item.

## Description

### Cross-Reference to Related Applications

This application claims priority pending United States Provisional Patent Application Serial No. 60/471,651 (Applicant Docket No. 2003P07266US), filed 19 May 2003.

### Background

Known systems provide for the generation, capture, storage, and analysis, interpretation, and visualization of medical images, lab values, and other clinical information. Such known systems do not, however, incorporate biochemical information such as molecular, genomic, genetic, protein, cellular, process, pathway, and/or biosignalling information and/or do not combine such biochemical information with molecular and/or traditional imaging in a clinically relevant fashion. The failure to incorporate such biochemical information retards a deeper understanding of biological processes, pathways, and signalling that would enable the emergence of a personalized, molecular, and/or genomic medicine that provides the opportunity for real prevention in terms of description of genetic predispositions, early diagnosis of molecular processes underlying early stages of distortion or diseases, and individualized therapeutic interventions. Certain exemplary embodiments of a system according to inventive principles addresses these and/or derivative problems.

### Summary

Among the many potential embodiments is a searchable electronic medical record system comprising: an interface for receiving data representing a search item; at least one repository associating a subject identifier with medical information specific to a particular subject comprising: genomic information, clinical data, and laboratory test results; and a search processor for searching said at least one repository to find medical information comprising said search item.

### Brief Description of the Drawings

The wide array of potential embodiments can be better understood through the following detailed description and the accompanying drawings in which:
**FIG. 1** is a block diagram of an exemplary embodiment of a system 1000;
**FIG. 2** is a block diagram of an exemplary embodiment of a method 2000; and
**FIG. 3** is a block diagram of an exemplary embodiment of an information device 3000.

### Definitions

When the following terms are used herein, the accompanying definitions apply:
**associating -** the act of defining a link and/or relationship between two or more elements.
**automatically** - acting or operating in a manner essentially independent of external influence or control. For example, an automatic light switch can turn on upon "seeing" a person in its view, without the person manually operating the light switch.
**biochemical information -** molecular, genomic, genetic, protein, cellular, process, pathway, and/or biosignalling information.
**bioluminescence imaging (BLI) -** a rapid and noninvasive functional imaging method that employs light-emitting reporters and external photon detection to follow biological processes in living animals in real time.
**calling procedure** - a procedure for enabling execution of another procedure in response to a received command or instruction.
**client** - an information device and/or process running thereon that requests a service of another information device or process running thereon (a "server") using some kind of protocol and accepts the server's responses. A client is part of a client-server software architecture. For example, a computer requesting the contents of a file from a file server is a client of the file server.
**clinical data -** data related to the history, diagnosis, and/or treatment of a patient.
**clinical trial -** a scientific test of the effectiveness and/or safety of a therapeutic agent using consenting human subjects.
c**ommunication protocol -** a set of rules governing transmitting and receiving data between computers and/or communications devices.
**complementary DNA or RNA -** DNA or RNA that is characterized by molecular complementarity, such as the capacity for precise pairing of purine and pyrimidine bases between strands of DNA and sometimes RNA such that the structure of one strand determines the other.
**compound -** a pure, macroscopically homogeneous substance consisting of atoms or ions of two or more different elements in definite proportions that cannot be separated by physical means. A compound usually has properties unlike those of its constituent elements.
**data -** numbers, characters, symbols etc., that are related to a subject. Data handling can be automated.
**database** - one or more structured sets of persistent data, usually associated with software to update and query the data. A simple database might be a single file containing many records, each of which is structured using the same set of fields. A database can comprise a map wherein various identifiers are organized according to various factors, such as identity, physical location, location on a network, function, etc.
**diagnosis code -** a code comprising one or more characters associated with a patient diagnosis, medical condition, or treatment, and that may possibly be used in reimbursement for a treatment. The diagnostic code is assignable based upon a reimbursement requirement by a third party. Alternatively, the diagnostic code is assignable based upon a need to analyze the utilization of medical resources. A set of diagnosis codes can conform to and/or be compatible with, for example, ICD (International Classification of Diseases) codes, 9th Edition, Clinical Modification, (ICD-9-CM), Volumes 1, 2 and 3; ICD-10, which is maintained and distributed by the U.S. Health and Human Services department; HCPCS (Health Care Financing Administration Common Procedure Coding System); NDC (National Drug Codes); CPT-4 (Current Procedural Terminology); Fourth Edition CDPN (Code on Dental Procedures and Nomenclature); SNOMED-RT "Systematicized Nomenclature of Medicine, Reference Terminology" by the College of American Pathologists; UMLS (Unified Medical Language System), by the National Library of Medicine; LOINC Logical Observation Identifiers, Names, and Codes; Regenstrief Institute and the Logical Observation Identifiers Names and Codes (LOINC®) Committee; Clinical Terms also known as "Read Codes"; DIN Drug Identification Numbers; Reimbursement Classifications including DRGs (Diagnosis Related Groups); CDT Current Dental Terminology; NIC (Nursing intervention codes); and Commercial Vocabulary Services (such as HealthLanguage by HealthLanguage Inc.); etc.
**DICOM -** Digital Imaging and Communications in Medicine. A standard developed by the American College of Radiology and the National Equipment Manufacturers Association to define the connectivity and communication protocols of medical imaging devices.
**diffuse optical tomography (DOT) -** an optical imaging method that uses photon density waves to probe the object (tissue). Photon sources and detectors are used in multiple geometric configurations around the object. Images of deep structures are formed by employing a mathematical reconstruction algorithm based on the solution of diffusion equations, under the assumption that photons have been scattered multiple times.
**disease code -** at least one character corresponding to a known and/or theorized disease.
**DNA -** a nucleic acid that carries the genetic information in the cell and is capable of self-replication and synthesis of RNA. DNA consists of two long chains of nucleotides twisted into a double helix and joined by hydrogen bonds between the complementary bases adenine and thymine or cytosine and guanine. The sequence of at least certain portions of the nucleotides determines individual hereditary characteristics. Also called deoxyribonucleic acid.
**DNA/RNA sequence -** a order of nucleotides in at least a portion of a nucleic acid.
**drug -** a substance used in the diagnosis, treatment, or prevention of a disease or as a component of a medication.
**electronic medical record system -** a computer based system for managing patient medical records.
**electronic patient medical record -** a computer based record of information related to a patient.
**executable application -** code or machine readable instructions for implementing predetermined functions including those of an operating system, healthcare information system, or other information processing system, for example, in response to a user command or input.
**executable procedure -** a segment of code (machine readable instruction), sub-routine, or other distinct section of code or portion of an executable application for performing one or more particular processes and may include performing operations on received input parameters (or in response to received input parameters) and provide resulting output parameters.
**experiment -** a test carried out under controlled conditions in order to discover an unknown effect or law, to test or establish a hypothesis, or to illustrate a known law.
**experimental protocol -** a description of a procedure used in an experiment.
**expressed sequence tags (EST) -** a plurality of sequence tagged sites derived from cDNAs.
**firmware** - machine-readable instructions that are stored in a read-only memory (ROM). ROM's can comprise PROMs, EPROMs, and EEPROMs.
**fluorescence in situ hybridization (FISH) -** a method of visualizing genes on a chromosome by means of fluorescence-labeled molecular probes.
**gene array -** a substrate upon which a collection of gene-specific nucleic acids have been placed at defined locations.
**gene array processing compatible protocol -** a protocol and/or markup language for data related to the processing of gene arrays.
**genomics -** a branch of biotechnology concerned with applying the techniques of genetics and molecular biology to the genetic mapping and DNA sequencing of sets of genes or the complete genomes of selected organisms using high-speed methods, with organizing the results in databases, and with applications of the data (as in medicine or biology).
**HL7** - Health Level 7, the ANSI standard for information interchange between foreign systems in the healthcare industry. The "7" refers to the fact that the protocol is designed to address the 7th layer of the International Standards Organization's Open System Interconnect model (the application layer).
**identifier** - a group of symbols that are unique to a particular entity, activity, and/or document. An identifier can be, for example, a medical record number. An identifier can be human and/or machine readable, such as for example, a number, an alphanumeric string, a bar code, an RFID, etc.
**image representative information -** data related to a rendering of an image.
**information -** data that has been organized to express concepts.
**information device** - a device capable of processing information, such as any general purpose and/or special purpose computer, such as a personal computer, workstation, server, minicomputer, mainframe, supercomputer, computer terminal, laptop, phone, and/or any equivalents thereof, etc.
**insurance company medical condition reimbursement code** - a diagnosis code utilized and/or recognized by an insurance company.
**interface -** a boundary across which two independent systems meet and act on or communicate with each other. To connect with or interact with by means of an interface.
**machine-readable media** - a memory readable by an information device.
**MAGE -** a unified description of, or markup language for, microarray expression data.
**medical -** of or relating to the study or practice of medicine.
**medical condition -** a physical, mental, emotional, visual, and/or dental status, mode, and/or circumstance.
**medical condition code -** at least one character corresponding to a known and/or theorized medical condition.
**medical image -** an image of a medical condition or medically-related data. Can be obtained via, for example, ultrasonography, radiography, magnetic resonance imaging, positron emission tomography, computed tomography, etc.
**medical record identifier -** an identifier for a patient medical record of a particular patient.
**messenger RNA (mRNA) -** an RNA produced by transcription that carries the code for a particular protein from the nuclear DNA to a ribosome in the cytoplasm and acts as a template for the formation of that protein.
**microarray technology -** a method for studying how large numbers of genes interact with each other and/or how the regulatory networks of a cell control numerous genes simultaneously. The method typically uses a robot to print tiny amounts of functional DNA samples precisely onto glass slides and/or a gene array. Researchers then attach fluorescent labels to DNA from the cell they are studying. The labeled probes are allowed to bind to complementary DNA strands on the slides. The slides are put into a scanning microscope that can measure the brightness of each fluorescent dot; brightness reveals how much of a specific DNA fragment is present, an indicator of how active it is.
**near-infrared fluorescence imaging (NIRF) -** a method for imaging of fluorescence photons in the near-infrared range (typically 600-900 nm). A fluorochrome is excited by a lower wavelength, and the emitted excitation is recorded as a slightly higher wavelength. NIRF imaging can be done in reflectance mode or in tomographic mode.
**network -** a coupling of two or more information devices to share resources (such as printers or CD-ROMs), exchange files, or allow electronic communications therebetween. Information devices on a network can be linked through various wireline or wireless media, such as cables, telephone lines, power lines, optical fibers, radio waves, light beams, etc.
**network interface** - **a** telephone, a cellular phone, a cellular modem, a telephone data modem, a fax modem, a wireless transceiver, an Ethernet card, a cable modem, a digital subscriber line interface, a bridge, a hub, a router, or other similar device.
**object -** a grouping of data, executable instructions or a combination of both or an executable procedure.
**optical imaging -** a family of methods for generating images by using photons of light in the wavelength range from ultraviolet to near-infrared, including the range of wavelengths visible to the human eye. The propagation of light through tissue is mainly influenced by absorption and scattering in the tissue itself or by a fluorescent contrast agent.
**patient identifier -** an identifier for a particular patient of a healthcare organization. A patient identifier might be a social security number, taxpayer ID number, national ID number, Medicare number, Medicaid number, medical insurance ID number, etc.
**patient medical information** - information relevant to the medical care and/or treatment of a patient, including real-time vital, biological, and/or physiological data, near real-time and/or prior history data relating to vital, biological, and/or physiological data, blood pressure parameters, ventilation parameters, vital sign parameters, blood oxygen concentration representative parameters, infusion pump parameters associated with fluid delivery, drip medication related parameters, blood gas parameters, insurance information, health care personnel information, health care organization information, billing information, family information, financial information, therapy information, drug information, and/or any equivalents thereof, etc.
**picture archiving and communication system (PACS) -** information devices and/or networks dedicated to the storage, retrieval, distribution, and presentation of medical images.
**processor** - a hardware, firmware, and/or software machine and/or virtual machine comprising a set of machine-readable instructions adaptable to perform a specific task. A processor acts upon information by manipulating, analyzing, modifying, converting, transmitting the information to another processor or an information device, and/or routing the information to an output device.
**proteomics -** a branch of biotechnology concerned with applying the techniques of molecular biology, biochemistry, and genetics to analyzing the structure, function, and interactions of the proteins produced by the genes of a particular cell, tissue, or organism, with organizing the information in databases, and with applications of the data (as in medicine or biology).
**protocol map -** a rendering of one or more relationships between protocols.
**record -** a collection of data elements. For example, a patient medical information database might contain records that have data elements stored in three fields: a name field, an address field, and a phone number field.
**render -** the act of making perceptible to a human, for example as data, commands, text, graphics, audio, video, animation, and/or hyperlinks, etc., such as via any visual and/or audio means, such as via a display, monitor, electric paper, ocular implant, speaker, cochlear implant, etc.
**repository -** a device or database in which data is stored.
**RNA -** a polymeric constituent of all living cells and many viruses, consisting of a long, usually single-stranded chain of alternating phosphate and ribose units with the bases adenine, guanine, cytosine, and uracil bonded to the ribose. The structure and base sequence of at least certain portions of RNA are determinants of protein synthesis and the transmission of genetic information. Also called ribonucleic acid.
**search item -** the content of a query, such as a database query.
**search processor -** a processor utilizable for searching for data, such as searching for data in a database of patient medical records.
**server -** an information device that provides some service for other information devices connected to it via a network. A common example is a file server, which has a local disk and services requests from remote clients to read and write files on that disk. A server can also provide access to resources, such as programs, shared devices, etc.
**test agent -** the subject of an experiment and/or a clinical trial. The subject may be, for example, a DNA/RNA sequence, compound, drug, and/or a treatment protocol, etc.
**transfer RNA (tRNA) -** a relatively small RNA that transfers a particular amino acid to a growing polypeptide chain at the ribosomal site of protein synthesis during translation. Also called adapter RNA and soluble RNA.
**treatment protocol -** a procedure for administration or application of one or more potential remedies to a patient or for a disease or injury.
**user -** an individual capable of utilizing a system for accessing patient information.
**user interface -** a device and/or program for rendering information to a user and/or requesting information from the user. A user interface can include textual, graphical, audio, video, animation, and/or haptic elements.

### Detailed Description

Among the many potential embodiments is a searchable electronic medical record system comprising: an interface for receiving data representing a search item; at least one repository associating a subject identifier with medical information specific to a particular subject comprising: genomic information, clinical data, and laboratory test results; and a search processor for searching said at least one repository to find medical information comprising said search item.

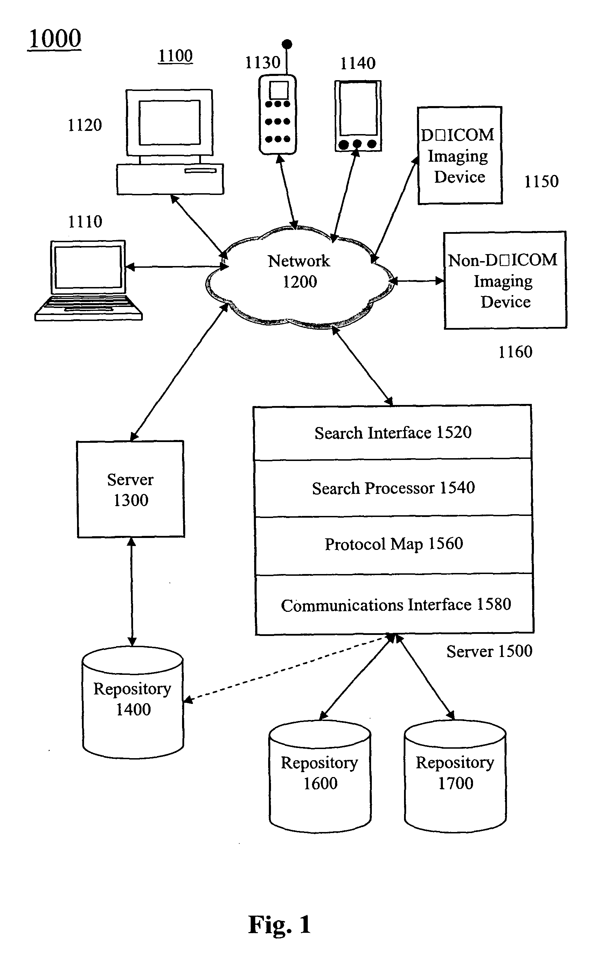

**FIG. 1** is a block diagram of an exemplary embodiment of a system 1000, which comprises any number of information devices 1100, such as laptop computer 1110, desktop computer 1120, cellphone-enabled computer 1130, personal digital assistant 1140, DICOM imaging device 1150, non-DICOM imaging device 1160, etc. Any of information devices 1100 are coupled to a network 1200, such as the Internet. Any of information devices 1100 may function as a client in a client-server relationship. Any information device may run a browser for rendering nformation obtained from the network. The browser may display the information in a native format, such as HTML, and/or can utilize a scripting language, plug-in, and/or helper application, such as Java, ActiveX, VisualBasic, QuickTime, Flash, Acrobat, etc.

Any number of servers 1300, 1500 are coupled to network 1200. Any of servers 1300, 1500 may function as a web server, data server, mail server, file server, PACS server, etc. Any number of repositories 1400 are coupled to server 1300. Any number of repositories 1400, 1600, 1700 are coupled to server 1500, comprises an electronic patient medical record, and/or associates medical information elements specific to a particular subject and/or subject identifier to form a logical Master Record and/or to store the medical information elements. Via the logical Master Record, any type of medical information element may be associated with any other appropriate type of medical information element.

The logical Master Record provided by server 1500 may provide data, metadata, and/or links to data and/or metadata that is and/or is associated with the medical information elements. When these medical information elements are obtained from repositories 1400, the Master Record may serve as a "database of databases". From another viewpoint, the Master Record can serve as an interface that unites multiple databases and/or repositories 1400, without necessarily importing and redundantly storing their data. Yet, the Master Record may comprise metadata of underlying data stored in the repositories 1400, that metadata generated by the Master Record and/or the repositories 1400. The generation of metadata by the Master Record may be preprogrammed and/or dynamically user-programmed.

Each of the various types of medical information elements may comprise subject information, subject identifiers, biochemical information, genomic information, proteomic information, clinical data, laboratory test results, medical image information, image representative information, medical condition data, medical condition information, patient information, patient identifiers, experiment data, experimental protocol information, clinical trial data, test agent information, experiment design information, grant proposal information, and/or related business data.

A subject may be, for example, a patient, animal, test agent, experiment trial, and/or clinical trial.

Genomic information may comprise DNA information, RNA information, complementary DNA or RNA information, transfer RNA (tRNA) information, messenger RNA (mRNA) information, Expressed Sequence Tags (EST), and/or a gene array expression.

Medical condition data may comprise (a) a medical condition code, (b) a disease code, (c) a diagnosis code, and/or (d) a insurance company medical condition reimbursement code.

A medical information element has a particular format. For example, medical image information may be formatted according to the DICOM standard, such as frequently found with certain systems involving magnetic resonance imaging (MRI), positron emission tomography (PET), computed tomography (CT), and/or single photon emission computed tomography (SPECT), etc. As another example, medical image information may be in a non-DICOM format, such as frequently found with certain systems utilized for near-infrared flourescence (NIRF), bioluminescence imaging (BLI), diffuse optical tomography (DOT), and/or fluorescence in-situ hybridization (FISH), optical imaging, etc.

As another example, medical condition information, such as that obtained when obtaining a patient history or physical examination, can be formatted as text and/or in a word processing format. Other medical information elements can be formatted in spreadsheet, graphical, animation, and/or voice formats, and/or in any standard, non-standard, and/or proprietary software format.

At least one server 1500 comprises a search interface 1520, a search processor 1540, a protocol map 1560, and a communications interface 1580. Search interface 1520 is adapted to receive data (such as a query) representing a search item(s) corresponding to one or more medical information elements. Search processor 1540 is adapted to format the received data for searching one or more repositories and/or to identify the type of medical information element(s) to be searched. Protocol map 1560 is adapted to link each type of medical information element to a compatible communications protocol of one or more repositories 1400, 1600, 1700 that store that type of medical information element, potentially in response to a determination of the type of medical information to be accessed during a search.

When needed, communications interface 1580 automatically adaptively selects the communications protocol from protocol map 1560, convert the formatted received data to the selected communications protocol, and/or transmit the converted formatted received to the appropriate repositories 1400, 1600, 1700.

For example, repository 1600 might store medical image information accessible via a DICOM compatible protocol and/or an HL7 compatible protocol. Search interface 1520 might receive, from a client information device 1100, data representing a query for SPECT medical images of the amygdala region of the human brain for a particular subject undergoing mild emotional stimulus during sleep. Search interface 1520 can format the data into a computer-readable query. Protocol map 1560 can provide a link between SPECT medical images and the DICOM compatible protocol and/or the HL7 compatible protocol. Communications interface 1580 might receive the formatted query from search interface 1520, and automatically consult protocol map 1560 to select the DICOM compatible protocol and/or the HL7 compatible protocol, convert the formatted query to the DICOM compatible protocol and/or the HL7 compatible protocol, and transmit the converted formatted query to repository 1600. Communications interface 1580 might also receive results of the query from repository 1600, convert the results to an Internet compatible communications format, such as TCP/IP, HTTP, FTP, etc., and/or transmit the results to a web server and/or a client information device 1100.

As another example, communication interface 1580 might access genomic information using, for example, a MAGE compatible protocol and/or another gene array processing compatible protocol. As yet another example, communications interface 1580 might access clinical data and/or laboratory test results information using an HL7 compatible protocol and/or a DICOM compatible protocol.

Using the Master Record, one can perform queries through the medical information in ways that were not previously possible (since prior to the invention much of this information was typically stored in hand-written lab notebooks that might have been dispersed over a range of locations). Furthermore since the information is stored in a standardized way, searches of the information can be performed at any time with minimal effort. For example, it would be possible to perform a search during the early research phase of an investigation (in the lab using animal models) or to perform a similar search during the final stages of a clinical trial to look for potential information that may not have been recognized earlier in the study. This supports the identification and selection of unique compounds that might prove most effective for a particular disease process.

By providing a data record that combines all information associated with, for example, a study, grant, study protocol, gene, chromosome, polymorphism, amino acid, protein, enzyme, cell type, physiology, system, molecule, substance, compound, drug, product, history, symptom, condition, disorder, disease, observation, test, test equipment, test result, report, analysis, diagnosis, clinical treatment, contra-indication, protocol, procedure, treatment response, subject, subject identifier, animal, patient, clinician, researcher, and/or organization, it is possible to display and perform advanced post-processing using information from multiple sources. For example, color patterns in a gene array may serve as pointers into DICOM images or other lab information.

The simultaneous display of multiple information sources might facilitate the association of information in advantageous ways. These associations currently may happen only through observations of researchers who are exposed to the relevant information at the same point in time. For example, the simultaneous display of relevant types of medical information elements might support diagnosis, compound selection, and/or analysis of a patient's pre-disposition to a particular treatment.

In certain embodiments, system 1000 provides a web interface that enables users external to a research team to contribute data, perform queries, and/or view experimental protocols and results. This collaboration is facilitated using controlled interfaces (e.g., login passwords, audit trails, access controls, encryption, digital certificates, etc.) that support HIPAA compliance, confidentiality, patient privacy, data security, and/or protection of intellectual property. This interface is used for interfaces between teams and/or individuals in the same institution as well as for teams and/or individuals from different institutions to collaborate.

For example, a histology laboratory across town and/or across the planet may "upload" images from slide preparations into the Master Record to be included in the experimental results and/or the Master Record can provide the images, metadata regarding the images, and/or a link to the images stored in, for example, a histology database. This may speed the research process (by using the query function to discover information that already exists), reduce costly redundant efforts (by avoiding repeating experiments whose results are already known), and broaden the scope of the research team by enabling more contributors to a given study.

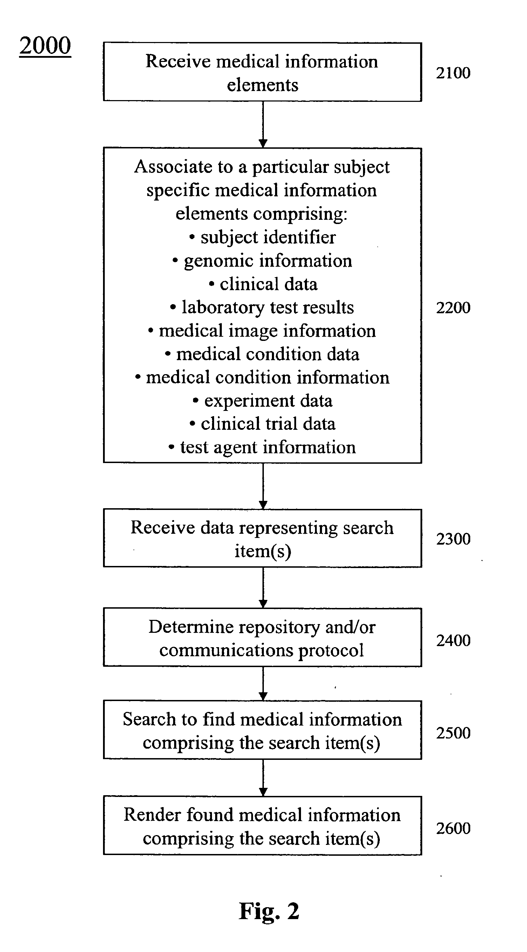

**FIG. 2** is a flow chart of an exemplary embodiment of a method 2000, which involves a medical information system such as system 1000 of **FIG. 1.** At activity 2100, medical information elements are received at one or more repositories. At activity 2200, medical information elements specific to a particular subject are associated to form a logical Master Record. In certain situations, the medical information elements are stored as the Master Record. In other situations, the Master Record merely links to the medical information elements. Both approaches can be used in the same Master Record.

At activity 2300, data is received representing a search item(s). For example, via a query interface, a user enters data for which they would like to search. This interface can facilitate search queries that are structured, unstructured, Boolean, and/or natural language, etc. At activity 2400, repositories to be searched and/or communications protocols to interface with those repositories are determined. At activity 2500, the repositories are searched to find medical information comprising the search item(s). At activity 2600, found medical information comprising the search item(s) is transmitted to a client information device for rendering to a user, such as via a browser.

In an exemplary search, for one or more given test agents that each have been proven to be effective for a given disease process, a query can be formed to identify which imaging and/or non imaging diagnostic process was most effective for each test agent.

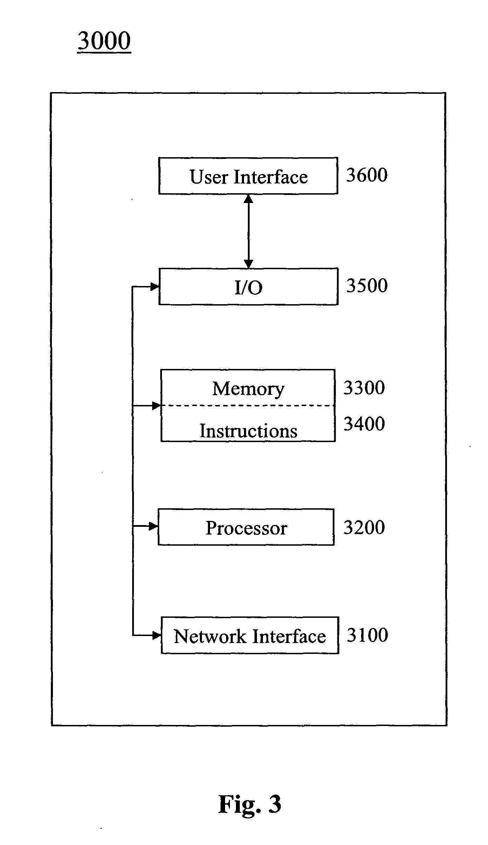

**FIG. 3** is a block diagram of an exemplary embodiment of an information device 3000, which can represent any information device 1100, server 1300, and/or server 1500, etc. of **FIG. 1.** Information device 3000 comprises any of numerous well-known components, such as for example, one or more network interfaces 3100, one or more processors 3200, one or more memories 3300 containing instructions 3400, one or more input/output (I/O) devices 3500, and/or one or more user interfaces 3600 coupled to I/O device 3500, etc. Via one or more user interfaces 3600, such as a graphical user interface, a user may provide medical information to a repository, generate a search query, and/or obtain results to a search query.

Still other embodiments will become readily apparent to those skilled in this art from reading the above-recited detailed description and drawings of certain exemplary embodiments.

## Claims

1. A searchable electronic medical record system, comprising:
an interface for receiving data representing a search item;
at least one repository associating a subject identifier with medical information specific to a particular subject comprising:
genomic information,
clinical data, and
laboratory test results; and
a search processor for searching said at least one repository to find medical information comprising said search item.

2. A system according to claim 1, wherein said at least one repository associates said subject identifier with medical information specific to a particular subject comprising:
image representative information,
a medical condition, and
an experiment or clinical trial.

3. A system according to claim 2, wherein said at least one repository associates said subject identifier with medical information specific to a particular subject comprising:
medical image representative information,
data identifying a medical condition, and
data identifying an experiment or clinical trial and
wherein said data identifying a medical condition comprises at least one of, (a) a medical condition code, (b) a disease code, (c) a diagnosis code, and (d) a insurance company medical condition reimbursement code.

4. A system according to claim 3, wherein said at least one repository associates said subject identifier with:
an experimental protocol comprising a description of said experiment or clinical trial, and
a test agent.

5. A system according to claim 4, wherein said test agent comprises at least one of, (a) a DNA/RNA sequence, (b) a compound, (c) a drug, and (d) a treatment protocol.

6. A system according to claim 3, wherein said at least one repository associates said subject identifier with proteomic information.

7. A system according to claim 1, wherein said subject is a patient and said subject identifier is a patient identifier.

8. A system according to claim 1, wherein said subject is at least one of, (a) patient, (b) an animal, (c) a test agent, and (d) an experiment or clinical trial.

9. A system according to claim 1, wherein said genomic information comprises at least one of, (a) DNA information, (b) RNA information, (c) complementary DNA or RNA information, (d) transfer RNA (tRNA) information (e) messenger RNA (mRNA) information, and (f) Expressed Sequence Tags (EST).

10. A system according to claim 1, wherein said genomic information comprises a gene array expression.

11. A system according to claim 1, wherein said at least one repository comprises an electronic patient medical record.

12. A searchable electronic medical record system, comprising:
an interface for receiving data representing a search item;
at least one repository associating medical information elements specific to a particular subject and comprising:
genomic information,
clinical data,
laboratory test results information, and
medical image information; and
a search processor for searching said at least one repository to find medical information comprising said search item.

13. A system according to claim 12, further comprising a communication interface for accessing said at least one repository using a communication protocol compatible with a type of said medical information.

14. A system according to claim 13, wherein said communication interface accesses said medical image information using a DICOM compatible protocol and accesses said genomic information using at least one of, (i) a MAGE compatible protocol and (ii) another gene array processing compatible protocol and accesses said clinical data and laboratory test results information using at least one of, (a) a HL7 compatible protocol, (b) a DICOM compatible protocol.

15. A system according to claim 13, wherein said communication interface automatically adaptively selects said communication protocol from predetermined protocol map information linking protocol and information type, in response to determination of said type of said medical information to be accessed during a search.

16. A searchable electronic medical record system, comprising:
an interface for receiving data representing a search item;
at least one repository associating medical information elements specific to a particular subject and comprising:
genomic information,
clinical data,
laboratory test results,
medical image information,
data identifying a medical condition,
data identifying an experiment or clinical trial, and
a test agent; and
a search processor for searching said at least one repository to find medical information comprising said search item.

17. A method for acquiring desired information from a searchable electronic medical record system, comprising the activities:
receiving data representing a search item;
associating a subject identifier with medical information specific to a particular subject comprising:
genomic information,
clinical data, and
laboratory test results; and
searching said at least one repository to find medical information comprising said search item in response to receiving said data representing said search item.

18. A method for acquiring desired information from a searchable electronic medical record system, comprising the activities:
receiving data representing a search item;
associating medical information elements specific to a particular subject and comprising:
genomic information,
clinical data,
laboratory test results information,
medical image information, and
data identifying a medical condition; and
searching said at least one repository to find medical information comprising said search item in response to receiving said data representing said search item.

19. A method for acquiring desired information from a searchable electronic medical record system, comprising the activities:
receiving data representing a search item;
associating medical information elements specific to a particular subject and comprising:
genomic information,
clinical data,
laboratory test results,
medical image information,
data identifying a medical condition,
data identifying an experiment or clinical trial, and
a test agent; and
searching said at least one repository to find medical information comprising said search item in response to receiving said data representing said search item.
